# EUROPEAN PATENT SPECIFICATION

(11) **EP 2 704 621 B1**
(45) Date of publication and mention of the grant of the patent: **09.06.2021**
(21) Application number: 12722914.4
(22) Date of filing: 25.04.2012
(51) Int. Cl.: A61B 5/00

(54) **OBJECT INFORMATION ACQUIRING APPARATUS AND CONTROL METHOD THEREOF**
VORRICHTUNG ZUR ERFASSUNG VON OBJEKTINFORMATIONEN UND STEUERVERFAHREN DAFÜR
APPAREIL D'ACQUISITION D'INFORMATIONS D'OBJET ET SON PROCÉDÉ DE COMMAND

(30) Priority: 02.05.2011 JP 2011102843
(43) Date of publication of application: 12.03.2014
(73) Proprietor: Canon Kabushiki Kaisha, Tokyo 146-8501 (JP)
(72) Inventor: TOKITA, Toshinobu, Ohta-ku Tokyo 146-8501 (JP)
(74) Representative: TBK
(86) International application number: PCT/JP2012/002834
(87) International publication number: WO 2012/150656

(56) References cited:
- EP-A1- 2 679 161
- EP-A1- 2 687 162
- WO-A1-2004/042382
- US-A- 5 718 228
- US-A1- 2003 055 360
- US-A1- 2007 010 742
- SERGEY A. ERMILOV ET AL: "Development of laser optoacoustic and ultrasonic imaging system for breast cancer utilizing handheld array probes", PROCEEDINGS OF SPIE, 1 January 2009 (2009-01-01), pages 717703-717703-10, XP55033734, DOI: 10.1117/12.812192 cited in the application

## Description

### [Technical Field]

The present invention relates to an object information acquiring apparatus and a control method thereof.

### [Background Art]

Attention has been focused on photoacoustic tomography (hereinafter will be referred to as "PAT") as a method of specifically imaging neovascularization which occurs due to cancer. PAT is a method including illuminating an object with illuminating light such as near infrared rays and receiving a photoacoustic wave generated from the inside of the object by means of an ultrasound probe, thereby imaging the photoacoustic wave. Such a photoacoustic apparatus is described in NPL 1.

NPL 1, however, is silent on a contact between an illuminating light emitting surface and the object. Therefore, it is possible that the illuminating light is emitted not only to the object but also into other space and, hence, there is room to improve the safety against illuminating light.

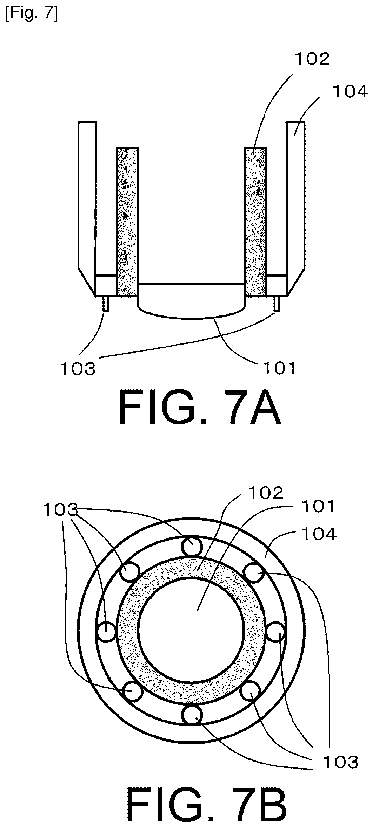

PTL 1 describes a technique for addressing this problem. Fig. 7 illustrates the configuration of the technique of PTL 1. Fig. 7A is a sectional view and Fig. 7B is a bottom view. In Fig. 7, an energy emitting surface 101 is a surface for contact with skin from which energy, such as light, is emitted. A support structure 102 fixes the energy emitting surface 101 and is housed in a housing 104 with contact sensors 103 intervening therebetween. The contact sensors 103 are each configured to detect a contact between the energy emitting surface 101 and non-illustrated skin and are disposed to circumscribe the energy emitting surface 101. Energy emission is stopped unless the contact between the contact sensors 103 and the skin is detected. By so doing, energy irradiation is conducted only when the energy emitting surface 101 is completely in intimate contact with the skin, thus improving the safety against energy irradiation.

### [Citation List]

### [Patent Literature]

[PTL 1]
Japanese Translation of PCT Application No. 2006-525036

### [Non Patent Literature]

[NPL 1]
S.A. Ermilov et al., Development of laser optoacoustic and ultrasonic imaging system for breast cancer utilizing handheld array probes, Photons Plus Ultrasound: Imaging and Sensing 2009, Proc. of SPIE vol. 7177, 2009. Prior art can be found e.g. in document WO 2004/042382 A1 disclosing a method and apparatus for non-invasive measurement of living body characteristics by photoacoustic, in prior art document EP 2 679 161 A1 according to Art. 54 (3) EPC disclosing an acousto-optical image generating and method, in prior art document EP 2 687 162 A1 according to Art. 54(3) EPC disclosing a photoacoustic image generating device and method. Furthermore, prior art can be found e.g. in document US 5 718 228 A disclosing an ultrasonic diagnostic apparatus .

### [Summary of Invention]

### [Technical Problem]

The conventional art, however, involves the following problems.

Since NPL 1 is silent on a contact between an illuminating light emitting surface and the object, it is possible that the illuminating light is emitted into a space other than object. For this reason, the safety against the illuminating light is not sufficient. Therefore, the operator or user has to exercise a considerable care to avoid emission of the illuminating light into such a space.

The technique described in PTL 1 addresses this problem. Specifically, the contact sensors for contact with skin are disposed around the illuminating light emitting surface to perform a control for stopping emission of the illuminating light unless the contact sensors detect a contact. However, the provision of a multiplicity of such contact sensors around the illuminating light emitting surface causes the system configuration to become larger in size. This leads to lowered operability for the operator or user.

The present invention has been made with the foregoing problems in view. An object of the present invention is to downsize the system configuration for photoacoustic tomography, thereby to improve the operability for the operator or user.

### [Solution to Problem]

The present invention provides an object information acquiring apparatus according to claim 1.

The present invention also provides a method for controlling an object information acquiring apparatus according to the respective claim.

### [Advantageous Effects of Invention]

According to the present invention, it is possible to downsize the system configuration for photoacoustic tomography, thereby to improve the operability for the operator or user.

Further features of the present invention will become apparent from the following description of examples and exemplary embodiments with reference to the attached drawings.

### [Brief Description of Drawings]

[Fig. 1]

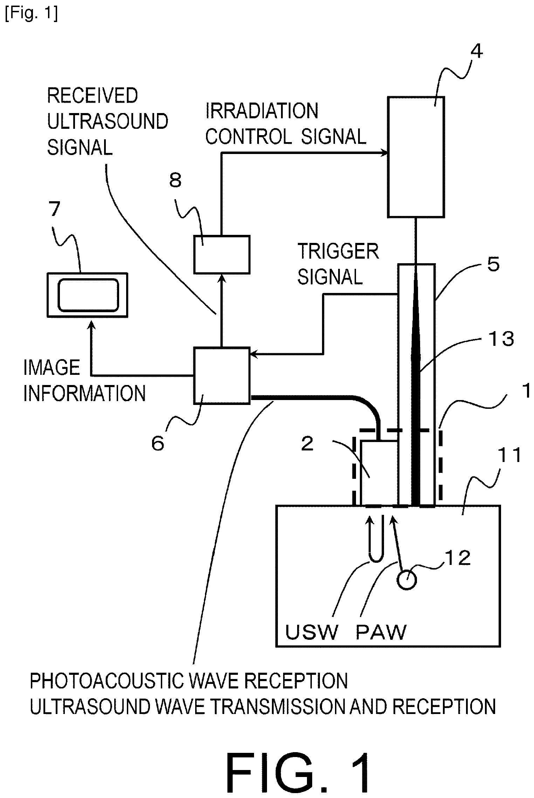

   Fig. 1 is a view illustrating a system configuration.
[Fig. 2]

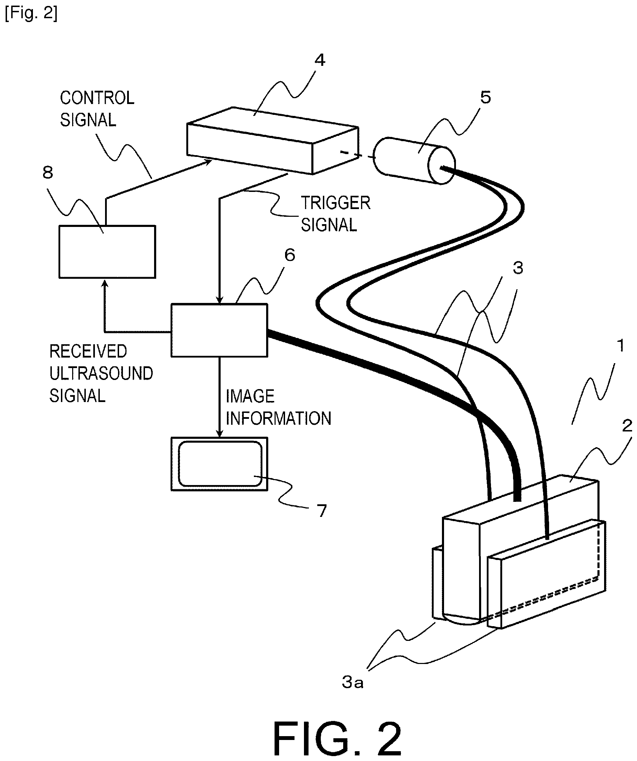

   Fig. 2 is a view illustrating a system configuration according to an Example useful for understanding the present invention.
[Fig. 3]

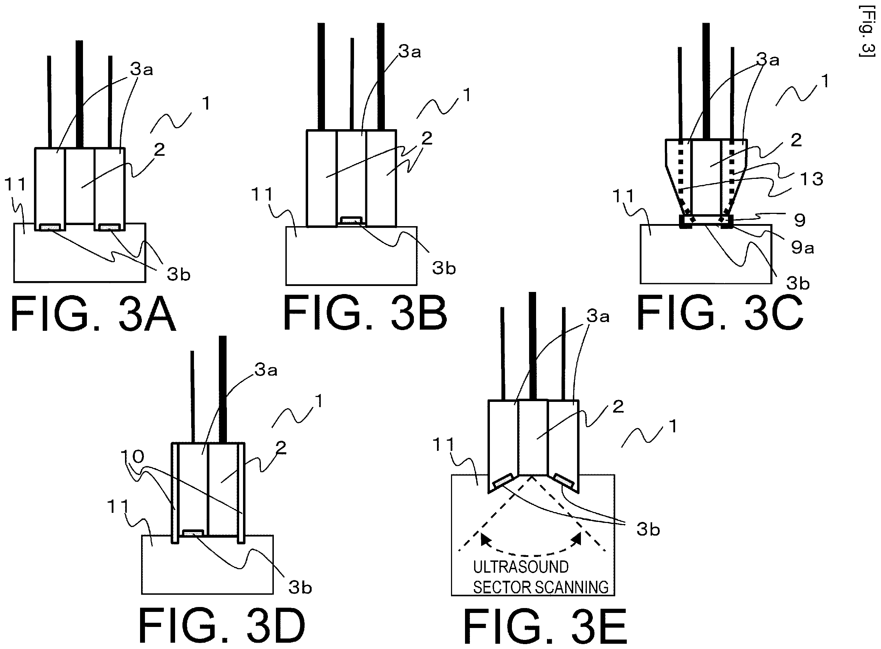

   Figs. 3A to 3E are views illustrating photoacoustic probe unit according to the Example useful for understanding the present invention.
[Fig. 4]

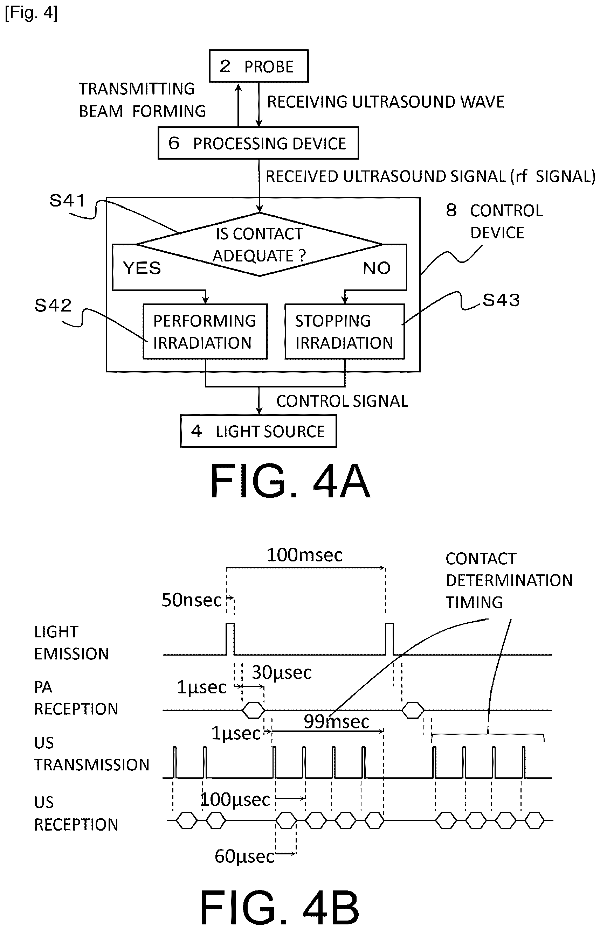

   Figs. 4A and 4B are charts illustrating control method according to the Example useful for understanding the present invention.
[Fig. 5]

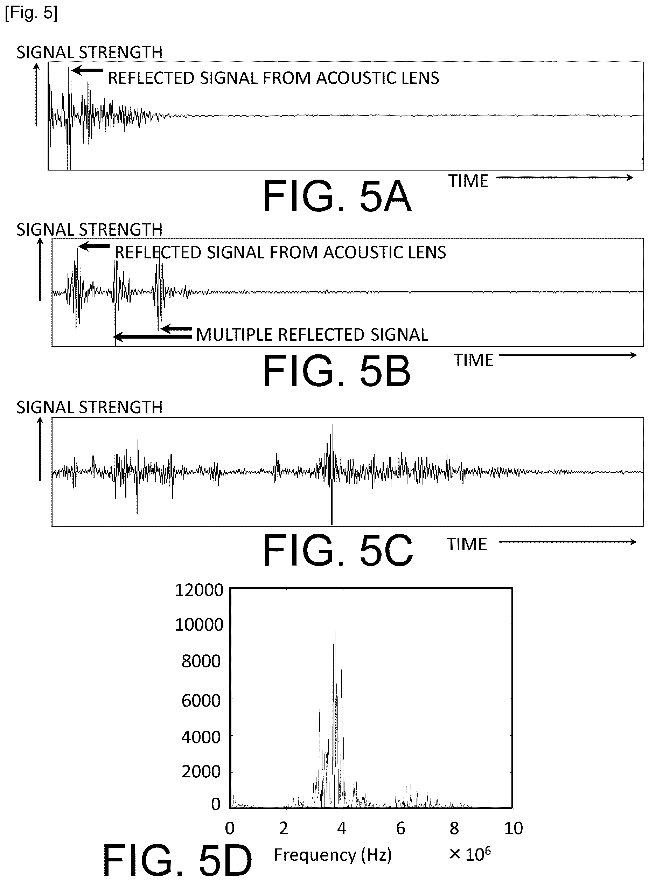

   Figs. 5A to 5D are charts illustrating method for ultrasound determination of contact according to the Example useful for understanding the present invention.
[Fig. 6]

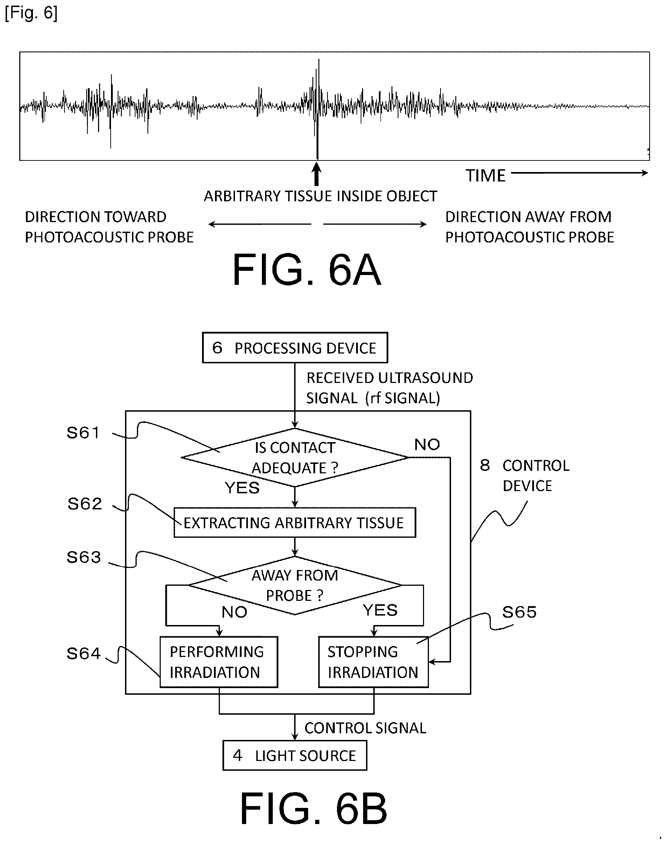

   Figs. 6A and 6B are charts illustrating control method according to an Embodiment of the present invention.
[Fig. 7]
   Figs. 7A and 7B are views illustrating the background art.

### [Description of Examples and Embodiments]

Hereinafter, preferred examples useful for understanding the present invention and embodiments of the present invention will be described with reference to the drawings. The present invention is applicable to apparatuses utilizing a photoacoustic effect for acquiring object information as image information by receiving an acoustic wave that is generated in an object by irradiating the object with light (electromagnetic wave) . (Such an acoustic wave is also called a "photoacoustic wave", a typical example of which is an ultrasound wave.) Such apparatuses are called "photoacoustic apparatuses". A photoacoustic apparatus according to the present invention is configured to utilize the ultrasound echo technique including transmitting an ultrasound wave to the object and then receiving a reflected wave resulting from reflection of the ultrasound wave inside the object to acquire object information as image information. Therefore, the apparatus according to the present invention can be called an object information acquiring apparatus serving both as a photoacoustic apparatus and an ultrasound echo apparatus.

When the apparatus of the present invention is regardedas the former, i.e., thephotoacoustic apparatus, the object information to be acquired means a distribution of generation sources of acoustic waves generated by irradiation with light, an initial sound pressure distribution inside the object, an absorbed optical energy density distribution or absorption coefficient distribution derived from the initial sound pressure distribution, or a concentration distribution of a substance forming a tissue. The concentration distribution of a substance is meant to include, for example, an oxygen saturation distribution and an oxidized-reduced hemoglobin concentration distribution.

When the object information acquiring apparatus is regarded as the latter, i.e., the ultrasound echo apparatus, the object information to be acquired is information reflecting an acoustic impedance difference between tissues inside the object.

The "acoustic wave" is typically an ultrasound wave and is meant to include elastic waves such as called a sound wave, an ultrasound wave, an acoustic wave, a photoacoustic wave, and a photoultrasound wave. The "light", as used in the present invention, is meant by electromagnetic waves including visible rays and infrared rays. Light of a specific wavelength is simply selected to meet a component to be measured by the object information acquiring apparatus.

The following description is directed to the object, though the object does not form part of the object information acquiring apparatus of the present invention. The object information acquiring apparatus according to the present invention is capable of diagnosing malignant tumors, vascular diseases, blood sugar levels, and the like of humans or animals, making follow-up of chemotherapy, and the like. Therefore, the object is assumed as a living body, specifically, a breast, finger, limb or the like of a human or animal. A light absorbing substance present inside the object is a substance having a relatively high absorption coefficient among substances present inside the object. For example, in cases where a human body is a subject of measurement, such light absorbing substances include oxidized or reduced hemoglobin and a blood vessel containing such hemoglobin, and a malignant tumor containing a number of angiogenesis.

Fig. 1 schematically illustrates a photoacoustic apparatus. A light source 4 emits illuminating light 13, and an illuminating optical system 5 irradiates an object 11 with the illuminating light 13. An irradiating end of the illuminating optical system is located adjacent to a probe 2, and the irradiating end and the probe form a photoacoustic probe unit 1. The illuminating light 13 permeates into the object and then a light absorbing substance 12 present inside the object generates a photoacoustic wave (PAW), which in turn is received by the probe 2. A processing device 6 performs amplification, digital conversion, detection and like processing of a photoacoustic signal received by the probe 2 in synchronization with an illuminating light emission trigger signal to create image information and causes a monitor 7 to display the image information. The processing device is equivalent to the "processor" defined by the present invention.

The probe 2 transmits and receives an ultrasound wave (USW) that is beamformed by the processing device 6 to and from the object 11 in order to acquire an ultrasound image. When the probe cannot receive any reflected wave from the object 11, a control device 8 determines that the photoacoustic probe unit 1 is out of contact with the object 11. When the probe has received a reflected wave from the object 11, on the other hand, the control device 8 determines that the photoacoustic probe unit 1 is in contact with the object 11. In this way, the control device determines the condition of contact with the object 11. The processing device 6 performs an irradiation control for causing the condition of contact with the object to be determined in the above-described manner and permitting irradiation during a contact condition while inhibiting irradiation during a non-contact condition.

The configuration described above makes it possible to downsize the photoacoustic probe unit 1 because the probe 2, which is a constituent element of the photoacoustic apparatus, can be utilized as a contact sensor. This leads to a simplified system configuration.

### <Example useful for understanding the invention>

In the Example useful for understanding the invention, the photoacoustic apparatus is described more specifically with reference to Fig. 2. The photoacoustic probe unit 1 comprises the probe 2 configured to receive a photoacoustic wave generated from the object (not shown) and the irradiating end for irradiating the object with illuminating light comprising near infrared rays. In one example, the irradiating end is provided with a bundled fiber 3. Though Fig. 1 does not show an illuminating optical system for guiding illuminating light from an irradiating end 3a of the bundled fiber to the object, it is possible to irradiate the object directly from the irradiating end 3a of the bundled fiber 3 or to provide any desired optical element such as a lens or a diffuser. The irradiating end is equivalent to the "light irradiating unit" defined by the present invention.

The light source 4 generates light (which comprises near infrared rays according to the present Example). The illuminating optical system 5 is configured to form the generated light into a beam having a beam radius and causes the light to become incident on the bundled fiber 3. A pulse laser, such as an Nd:YAG laser or an alexandrite laser, is used as the light source 4. Alternatively, use may be made of a Ti:Sa laser or OPO laser which uses Nd:YAG laser light as excitation light.

The illuminating light is partially branched to measure light emission of the light source 1 by means of a photodiode (not shown). The output of the photodiode is used as a trigger signal. When the trigger signal is inputted to the processing device 6, the probe 2 acquires a photoacoustic signal. Thereafter, the photoacoustic signal is subjected to amplification, digital conversion, detection and the like to create image information, which in turn is displayed on the monitor 7. The trigger signal is not limited to a signal generated by the photodiode, but it is effective to use a method of synchronizing light emission of the light source 4 to an input trigger for the processing device 6 by using a signal generator.

The probe 2 transmits and receives an ultrasound wave which is beamformed by the processing device 6 to and from the object in order to acquire an ultrasound image. The ultrasound wave is used not only in acquiring the ultrasound image but also in determining a contact between the photoacoustic probe unit 1 and the obj ect are in contact with each other. An array of detecting elements such as PZTs or CMUTs, for example, can be used as the probe 2.

When the reflected wave from the object cannot be received, the control device 8 determines that the photoacoustic probe unit 1 is out of contact with the object. When the reflected wave from the object has been received, on the other hand, the control device determines that the photoacoustic probe unit 1 is in contact with the object. In this way, the control device 8 determines the condition of contact with the object and transmits an irradiation control signal to the light source 1. The control device is equivalent to the "controller" defined by the present invention. The processing device and the control device can be implemented by using an information processor for example. The information processing device may perform different processing operations by using respective circuits dedicated thereto or may be implemented in the form of a program for operating a computer having a CPU or the like.

With reference to Fig. 3, description is made below of the configuration of the photoacoustic probe unit 1.

When an ultrasound wave transmitting and receiving portion of the probe 2 is acoustically matched with the object 11, the irradiating end of the photoacoustic probe unit 1 for emitting illuminating light 13 needs to be in contact with the object. Alternatively, another member located around the irradiating end needs to be in contact with the object. By so doing, it becomes possible to considerably reduce the illuminating light 13 emitted from between the photoacoustic probe unit 1 and the object 11. With reference to Figs. 3A to 3E, different configurations for the respective cases are described below.

The photoacoustic probe unit 1 shown in Fig. 3A has a configuration in which the probe 2 is sandwiched between irradiating ends 3a of the bundled fiber from outside in such a manner that the ultrasound wave transmitting and receiving surface of the probe 2 is positioned more apart from the object 11 than irradiating ends 3b. Alternatively, the probe 2 may be circumscribed by the irradiating ends 3a from outside. By thus providing a difference in height between the probe 2 and the illuminating light irradiating ends 3b, the illuminating light emitting ends 3b are brought into contact with the object 11 as long as the ultrasound wave transmitting and receiving surface of the probe is in contact with the object 11.

The photoacoustic probe unit 1 shown in Fig. 3B has a configuration in which an irradiating end 3a of the bundled fiber is sandwiched between probes 2 from outside in such a manner that an irradiating end 3a of the bundled fiber is positioned coplanar with the probes 2 with respect to the object 11 or more apart from the object than the probes. Alternatively, the irradiating end 3a may be surrounded by the probes 2 from outside. By thus providing a difference in height between the probes 2 and the illuminating light irradiating end 3b, the illuminating light irradiating end 3b or the probes around the irradiating end are brought into contact with the object 11 as long as the ultrasound wave transmitting and receiving surfaces of the probes are in contact with the object 11. That is, the object is irradiated with light from the irradiating end of the photoacoustic probe unit, while the probe is acoustically matched with the object. The contact condition assumed at that time is a first contact condition which is an adequate contact condition.

The photoacoustic probe unit 1 shown in Fig. 3C has a configuration in which the ultrasound wave transmitting and receiving surface of the probe 2 is provided with an acoustically matching member 9 so that the illuminating light 13 emitted from irradiating ends 3a of the bundled fiber passes toward the irradiating end 3b through the acoustically matching member 9 to illuminate the object 11. Preferably, side surfaces of the acoustically matching member 9 and that surface of the acoustically matching member which faces the object except the irradiating end 3b are provided with a light-shielding material 9a (i.e., reflecting material or absorbing material) for blocking the illuminating light 13 in order to prevent the illuminating light 13 from emitting from a portion other than the emitting end 3b. The acoustically matching member 9 comprises a material having a high transmissive property with respect to sound and illuminating light 13, preferred examples of which include polymethylpentene and urethane. The acoustically matching member 9 may serve to diffuse the illuminating light 13. By thus providing a difference in height between the ultrasound wave transmitting and receiving surface of the probe 2 and the illuminating light irradiating end 3b with respect to the object 11, the illuminating light irradiating end 3b is brought into contact with the object 11 when the probe 2 is acoustically matched with the object 11 by means of the acoustically matching member 9.

The photoacoustic probe unit 1 shown in Fig. 3D has a configuration in which both the probe 2 and the irradiating end 3a are sandwiched by a light-shielding walls 10 from outside in such a manner as to provide unevenness on that surface of the photoacoustic probe unit 1 which faces the object, though the ultrasound wave transmitting and receiving surface of the probe 2 and the illuminating light emitting end 3b are of the same height. Alternatively, the photoacoustic probe unit may be wholly surrounded by the light-shielding wall 10 from outside. The light-shielding wall is positioned so as not to interfere with ultrasound wave transmission and reception and irradiation with light. This feature allows the illuminating light irradiating end 3b or the light-shielding wall 10 around the irradiating end 3b to be brought into contact with the object 11 as long as the ultrasound wave transmitting and receiving surface of the probe 2 is in contact with the object 11.

As a variation of the photoacoustic probe unit 1 shown in Fig. 3A, an irradiating end 3b may be tapered or provided with a curvature, as shown in Fig. 3E. This feature allows the object 11 to be easily fitted into the recess defined between the tapered portions of the irradiating end 3b. Therefore, the illuminating light irradiating end 3b is brought into contact with the object 11 more reliably as long as the ultrasound wave transmitting and receiving surface of the probe 2 is in contact with the object 11. Use of probe 2 capable of sector scanning enables ultrasound wave transmission and reception by the probe 2 for determination of contact with the object 11 to be performed in a sector scanning manner without interference with the irradiating end 3b, thus making it possible to realize determination of contact with a broader area.

Though in all the configurations shown in Figs. 3A to 3E the surface for contact with the object has corners each right-angled or acute-angled, such corners are preferably rounded for actual use.

With reference to Fig. 4A, description is made below of a control method carried out by the control device 8.

Fig. 4A is a flowchart illustrating an irradiation control performed by the control device 8. The processing device 6 performs transmit beamforming on a vibrator in the probe 2 for acquiring an ultrasound image. Then, the processing device 6 performs reception beamforming on a signal received by the probe 2 from the object to acquire the ultrasound image. On the other hand, the processing device 6 transmits a received ultrasound signal (rf signal) to the control device 8.

In turn, the control device 8 starts a control process. Initially, the control device determines from the rf signal whether or not the contact between the photoacoustic probe unit 1 and the object is adequate (step S41).

If the contact condition is determined as being adequate (S41 = YES), the control device permits irradiation (step S42).

If the contact condition is determined as being inadequate (S41 =NO), on the other hand, the control device transmits an irradiation control signal to the light source 4 to stop irradiation with the illuminating light (step S43).

Either a method including opening and closing an internal shutter of the light source 4 or a method including controlling an internal trigger signal (generated by a flash lamp and a Q switch) is effective for the irradiation control over the light source 4. While the control signal from the control device 8 has been described as a signal for the irradiation control over the light source 1, there is no limitation to this feature. For example, it is possible that an external shutter is provided between the light source 1 and the illuminating optical system 2 while the opening and closing of the shutter is controlled by such a control signal.

With reference to the timing chart of Fig. 4B, description of irradiation control timing is made below. In Fig. 4B, photoacoustic signal reception (PA reception) is to acquire signals for a predetermined time period (e.g., 30 microseconds) in response to light emission serving as a trigger. The light emission interval is determined from the emission frequency of the light source 4. When the emission frequency is 10 Hz for example, the light emission interval is 100 msec. That is, the time period from the end of photoacoustic signal acquisition to the next light emission is not less than 99 msec. During this time period, the irradiation control described using the flowchart of Fig. 4A is performed. When the irradiation is stopped because of an inadequate contact, the irradiation is suspended until a contact is made. Immediately after the contact has been made, the irradiation control signal is transmitted to the light source 4 for causing the light source to perform irradiation. In cases where the light source 4 is controlled by the flash lamp and the Q switch, irradiation is performed at the next light emission timing in synchronization with the flash lamp.

With reference to Fig. 5, description is made below of determination of contact between the photoacoustic probe unit 1 and the object by means of the probe 2.

Fig. 5 shows a waveform received by the probe 2 (rf signal resulting after delay and sum). Fig. 5A shows a waveform received when the probe 2 is not in contact with any matter. Fig. 5B is a waveform received when only sonar gel is attached to the surface of the probe 2. Fig. 5C shows a waveform received when the surface of the probe 2 is in contact with the object via the sonar gel. The sonar gel functions as an acoustically matching agent and forms a matching layer. Further, the probe is in contact with an acoustic lens which functions as an acoustic member in receiving an ultrasound wave.

In Figs. 5A and 5B, signals are detected from a range down to a predetermined depth which is coincident with or adjacent to the boundary between the acoustic lens and air, though the probe is not in contact with any matter actually (i.e., inair). Specifically, reflected signals from a range from the vibrator in the probe 2 down to the acoustic lens are observed in Fig. 5A. In Fig. 5B, in addition to the reflected signals from the range down to the acoustic lens, multiple reflected signals from the acoustic lens and from the matching layer are also observed. From a position deeper than the predetermined depth, only noise-level signals of the probe 2 and processing device 6 are observed. Therefore, in the cases of Figs. 5A and 5B, it can be determined that no reflected signals are acquired from the object.

In Fig. 5C, on the other hand, such reflected signals (from positions coincident with the acoustic lens and the matching layer) are relatively small as compared with Figs. 5A and 5B. Further, ultrasound signals are received from a deeper position than the above-described predetermined depth (from which reflected waves are received later). Therefore, it can be determined that in the case of Fig. 5C an ultrasound wave has permeated into a substance having a relatively small acoustic impedance difference (e.g., the object in a state of being acoustically matched with the probe), whereas in the cases of Figs. 5A and 5B the acoustic impedance has varied largely because of an ultrasound wave permeating into air.

In this way, in the case where signals from the predetermined depth which is coincident with or adjacent to the boundary of the photoacoustic probe unit are present and larger than signals from the object acoustically matched with the photoacoustic probe unit or multiple reflection takes place, it can be determined that the photoacoustic probe unit 1 is out of contact with the object. In the case where there are no signals or small signals from the predetermined depth, it can be determined that the photoacoustic probe unit is in contact with the obj ect.

Whether or not signals from the predetermined depth are present can be determined by determining whether or not an rf signal is acquired which is several times as large as the S/N ratio of the probe 2 and processing device 6. For example, if there is a signal from a depth of 10 mm which is twice or more times, preferably three or more times as large as the S/N ratio, the probe 2 can be determined as being in contact with the object. There is no limitation to the depth of 10 mm. It is preferable to determine signals from plural points. There is no limitation to the value of twice or more times or three or more times as large as the S/N ratio which is used as a criterion for determination.

The presence or absence of a reflected signal from the range from the vibrator in the probe 2 to the matching layer or the acoustic lens can be determined from the presence or absence of a repeated signal within the range down to the predetermined depth on which attention is focused here. For example, the presence or absence of such a reflected signal can be determined by conducting Fourier transform of an rf signal from the range down to a depth of 5 mm and determining the presence or absence of a predetermined frequency component. Assuming that the average acoustic velocity from the vibrator of the probe 2 to the acoustic lens is 2000 m/s and the thickness is 0.25 mm, a frequency component corresponding to a round-trip distance of 0.5 mm which is an ultrasound propagation distance is detected (200 m/s/0.5 mm = 4 MHz). This can be seen from a peak of the rf signal having been subjected to Fourier transform which appears at around 3.8 MHz as shown in Fig. 5D resulting from Fourier transform of Fig. 5A.

It is needless to say that the depth specified here varies depending on the structure of the photoacoustic probe unit, the thickness of the object and the like and has to be appropriately established to meet such conditions.

The frequency component to be determined may be selected from a range, for example, a range from 3 MHz to 4 MHz in the example shown in Fig. 5D. Since the frequency specified here is attributable to the structure of the probe, there is no limitation to this value. Use may be made of any other method of detecting multiple reflected signals.

Alternatively, it is possible to determine that the probe 2 and the object are out of contact with each other when only the noise components inherent in the probe 2 and processing device 6 are detected from rf signals from a depth of not less than 7 mm for example which have been subjected to Fourier transform. When a frequency component other than those corresponding to noises is detected, the probe 2 and the object may be determined as being in contact with each other.

As described above, by conducting determination using at least one of a signal from the predetermined depth and a reflected signal from the range from the vibrator in the probe 2 to the matching layer or the acoustic lens, determination of contact between the probe 2 and the object becomes possible. As described with reference to Fig. 3, when the probe 2 of the photoacoustic probe unit 1 is acoustically matched with the object, the illuminating light irradiating end or another member located around the irradiating end is in contact with the object. When the illuminating light is emitted against the object from the photoacoustic probe unit 1 assuming such a condition, it becomes possible to considerably reduce the illuminating light emitted from between the photoacoustic probe unit 1 and the object.

While the determination of contact using rf signals obtained after having been subjected to phasing and adding has been described herein, a similar method may be applied to ultrasound wave transmission and reception relying upon a single element. Likewise, a similar method may be applied to signals obtained after having been subjected to detection. Though the determination of contact is preferably conducted based on all the received ultrasound signals (rf signals), the determination of contact is possible based on at least ultrasound signals received at opposite ends or corners of the probe 2 (particularly in the case of a two-dimensional array probe) is possible.

While the method having been described carries out ultrasound wave transmission and reception for both of the ultrasound image acquisition and the determination of contact, ultrasound wave transmission and reception may be carried out only for the determination of contact without acquiring an ultrasound image. In such a case, transmit and reception beamforming is unnecessary and the determination of contact between the photoacoustic probe unit 1 and the object can be carried out based on received signals acquired by driving a plurality of vibrators in the probe 2.

As described above, the probe 2, which is a constituent element of the photoacoustic apparatus, can be utilized as a contact sensor and, for this reason, the photoacoustic probe unit 1 can be downsized. When the number of ultrasound transmission and reception beams used in the determination of contact is increased, multiple determination becomes possible, which leads to improved safety. Further, since the determination of contact and the ultrasound image acquisition can be performed at the same time, illuminating light irradiation control and data acquisition can be performed efficiently.

In the present Example, description has been made of the configuration utilizing ultrasound wave transmission and reception by the probe 2 serving as one of the contact sensors and the control method thereof. It is natural that a separate contact sensor may be provided for multiple safety measures. In contrast to multiple safety measures using plural types of contact sensors without the probe 2, the present Example can use the probe 2 as one contact sensor and hence can realize downsizing of the whole photoacoustic probe unit 1.

### <Embodiment>

In the Example useful for understanding the invention, description has been made of the determination of contact between the photoacoustic probe unit 1 and the object carried out by transmitting and receiving ultrasound waves to and from the object by means of the probe 2, as well as the irradiation control. In the Embodiment, description is made of a method of irradiation control by foreseeing the photoacoustic probe unit 1 in a state of being about to separate from the object based on the result of repeated ultrasound wave transmission and reception.

Like Fig. 5C, Fig. 6A shows an ultrasound signal (rf signal) received when the probe 2 is in contact with the object. As indicated by arrow in Fig. 6A, an rf signal from an arbitrary tissue inside the object is observed.

The irradiation control is described below with reference to the flowchart of Fig. 6B.

According to the process for determination of contact described in the Example useful for understanding the invention, the control device 8 determines from the rf signal whether or not the contact between the photoacoustic probe unit 1 and the object is adequate (step S61).

If the contact is determined as being adequate (S61 = YES), the control device extracts an rf signal from an arbitrary tissue inside the object (step S62). Preferably, the "arbitrary tissue" is a tissue expansively present in the object like a subcutaneous fat layer for example. If the contact is determined as being inadequate (S61 = NO), the control device stops irradiation (step S65).

Subsequent to step S62, the control device determines a movement of the tissue relative to the photoacoustic probe unit 1. Thereafter, the control device determines whether or not the movement of the tissue is in the direction away from the photoacoustic probe unit (step S63).

If the time required for transmission and reception becomes shorter or remains the same, the tissue is considered as moving in the direction toward the photoacoustic probe unit indicated in Fig. 6A or remaining stationary. That is, the control device does not determine that the tissue is moving in the direction away from the photoacoustic probe unit (S63 = NO) and then permits irradiation with illuminating light (step S64).

On the other hand, if the time required for transmission and reception becomes longer, i.e., if the tissue is moving in the direction away from the photoacoustic probe unit indicated in Fig. 6A, the control device foresees that the photoacoustic probe unit 1 will separate from the object soon (S63 = YES) and then performs an irradiation control for stopping irradiation in advance (step S65). In a more preferable arrangement, a temporal differential of the amount of the movement, i.e., a threshold value of the velocity of the movement, is provided and an irradiation control for stopping irradiation is performed when the velocity exceeds the threshold value.

As described above, the present embodiment is capable of performing irradiation control by foreseeing the contact condition between the photoacoustic probe unit 1 and the object and hence further improves the safety.

While the present invention has been described with reference to examples useful for understanding the invention and exemplary embodiments, it is to be understood that the invention is not limited to the disclosed exemplary embodiments but by the scope of the following claims.

## Claims

1. An object information acquiring apparatus comprising:
a photoacoustic probe unit (1) including a light irradiating unit (3) configured to guide light (13) from a light source (4) to irradiate an object (11), and a probe (2) configured to receive a photoacoustic wave generated from the object irradiated with the light by the light irradiating unit, the probe (2) being configured to transmit an ultrasound wave to the object (11) and to receive a reflected wave of the transmitted ultrasound wave;
a processor configured to create image information on an internal part of the object based on the photoacoustic wave received by the probe; and
a controller (8) configured to control the irradiation with the light from the light irradiating unit,
**characterized in that**
the controller (8) is configured to determine whether or not the photoacoustic probe unit (1) is in contact with the object (11) in such a manner that the probe (2) is acoustically matched with the object (11) by using the reflected wave;
the controller (8) is further configured to determine a movement of a tissue in the object (11) based on a time required for transmission and reception of the ultrasound wave to the tissue;
the controller (8) is further configured to enable the irradiation with the light from the light irradiating unit (3) to be performed when the photoacoustic probe unit is determined as being in contact with the object (11) and the tissue is moving in the direction toward the photoacoustic probe unit (1) or stationary relative to the photoacoustic probe unit (1);
the controller (8) is further configured to stop the irradiation with the light from the light irradiating unit (3) to be performed when the photoacoustic probe unit (1) is determined as not being in contact with the object (11), or when the photoacoustic probe unit (1) is determined as being in contact with the object (11) and the tissue is moving in the direction away from the photoacoustic probe unit.

2. The object information acquiring apparatus according to claim 1, wherein the light irradiating unit is located more outwardly than the probe.

3. The object information acquiring apparatus according to claim 1, wherein the probe is located more outwardly than the light irradiating unit, and the light irradiating unit has an irradiating end which is positioned coplanar with an ultrasound wave transmitting and receiving surface of the probe.

4. The object information acquiring apparatus according to claim 1, wherein an ultrasound wave transmitting and receiving surface of the probe is provided with an acoustically matching agent, and an irradiating end of the light irradiating unit is in contact with the acoustically matching agent.

5. The object information acquiring apparatus according to claim 1, wherein the photoacoustic probe unit further includes a light-shielding wall located outwardly of the probe and the light irradiating unit, and an ultrasound wave transmitting and receiving surface of the probe and an irradiating end of the light irradiating unit are positioned more apart from the object than an end of the light-shielding wall.

6. The object information acquiring apparatus according to claim 1, wherein the controller is configured to determine that the probe is not acoustically matched with the object in a case where the reflected wave acquired from a position coinciding with a boundary between the photoacoustic probe unit and the object is larger than a signal acquired from the object in a state of being acoustically matched.

7. The object information acquiring apparatus according to claim 1, wherein the controller is configured to determine that the probe is acoustically matched with the object in a case where the reflected wave acquired from a position coinciding with an internal part of the object is larger than noises generated from the probe and the processor.

8. The object information acquiring apparatus according to claim 1, wherein the controller is configured to determine that the probe is acoustically matched with the object in a case where the reflected wave acquired from a position coinciding with an internal part of the object contains a frequency component other than frequency components corresponding to noises generated from the probe and the processor.

9. The object information acquiring apparatus according to claim 1,
wherein the probe is configured to transmit the ultrasound wave repeatedly to the object and to receive the reflected wave thereof, and
the controller is configured to extract a signal corresponding to the tissue inside the object from the reflected wave, and then to determine that the photoacoustic probe unit is moving in the direction away from the object in a case where a longer time is required for later ultrasound wave transmission and reception to acquire the signal thus extracted.

10. The object information acquiring apparatus according to claim 1, wherein the controller is configured to stop the irradiation with light from the light irradiating unit in a case where the probe unit and the object are relatively moving in a direction away from each other and a velocity at which the probe unit and the object are relatively moving away from each other exceeds a threshold value.

11. The object information acquiring apparatus according to claim 1, wherein the light irradiating unit is configured to perform the light irradiation at periodical irradiation timings.

12. The object information acquiring apparatus according to claim 1,
wherein the light irradiating unit is configured to guide the light from the light source to irradiate, from an illuminating light emitting end (3b) of the light irradiating unit, the object, and the probe is configured to receive, at a receiving surface, the photoacoustic wave generated from the object irradiated with the light by the light irradiating unit, and
wherein the photoacoustic probe unit further includes a light-shielding wall located outwardly of the probe and the light irradiating unit,
the illuminating light emitting end of the light irradiating unit and the receiving surface of the probe are of a same height; and
an end of the light-shielding wall projects beyond the height of the illuminating light emitting end and the receiving surface to provide unevenness on a surface of the photoacoustic probe unit comprising the illuminating light emitting end and the receiving surface.

13. A method for controlling an object information acquiring apparatus having:
a photoacoustic probe unit (1) including a light irradiating unit (3) configured to guide light (13) from a light source (4) to irradiate an object (11), and a probe (2) configured to receive a photoacoustic wave generated from the object irradiated with the light by the light irradiating unit, the probe (2) being configured to transmit an ultrasound wave to the object (11) and to receive a reflected wave of the transmitted ultrasound wave;
a processor configured to create image information on an internal part of the object based on the photoacoustic wave received by the probe; and
a controller (8) configured to control the irradiation with the light from the light irradiating unit,
**characterized in that**
the method comprises the steps of:
determining, by the controller (8), whether or not the photoacoustic probe unit (1) is in contact with the object (11) in such a manner that the probe (2) is acoustically matched with the object (11) by using the reflected wave;
determining, by the controller (8), a movement of a tissue in the object (11) based on a time required for transmission and reception of the ultrasound wave to the tissue;
enabling, by the controller (8), the irradiation with the light from the light irradiating unit (3) to be performed when the photoacoustic probe unit is determined as being in contact with the object (11) and the tissue is moving in the direction toward the photoacoustic probe unit (1) or stationary relative to the photoacoustic probe unit (1);
stopping, by the controller (8), the irradiation with the light from the light irradiating unit (3) to be performed when the photoacoustic probe unit is determined as not being in contact with the object (11), or when the photoacoustic probe unit (1) is determined as being in contact with the object (11) and the tissue is moving in the direction away from the photoacoustic probe unit.

## Patentansprüche

1. Objektinformationserhaltevorrichtung mit:
einer fotoakustischen Messkopfeinheit (1) mit einer Lichtbestrahlungseinheit (3), die konfiguriert ist, Licht (13) von einer Lichtquelle (4) zu leiten, um ein Objekt (11) zu bestrahlen, und einem Messkopf (2), der konfiguriert ist, eine fotoakustische Welle, die von dem Objekt, das mit dem Licht durch die Lichtbestrahlungseinheit bestrahlt wird, erzeugt wird, zu empfangen, wobei der Messkopf (2) konfiguriert ist, eine Ultraschallwelle auf das Objekt (11) zu transmittieren und eine reflektierte Welle der transmittierten Ultraschallwelle zu empfangen;
einer Verarbeitungseinheit, die konfiguriert ist, eine Bildinformation über einen inneren Teil des Objekts basierend auf der fotoakustischen Welle, die durch den Messkopf empfangen wird, zu erzeugen; und
einer Steuerungseinheit (8), die konfiguriert ist, die Bestrahlung mit dem Licht von der Lichtbestrahlungseinheit zu steuern,
**dadurch gekennzeichnet, dass**
die Steuerungseinheit (8) konfiguriert ist, unter Verwendung der reflektierten Welle zu bestimmen, ob die fotoakustische Messkopfeinheit (1) in Kontakt mit dem Objekt (11) in solch einer Weise ist, dass der Messkopf (2) akustisch an das Objekt angepasst ist oder nicht;
die Steuerungseinheit (8) ferner konfiguriert ist, eine Bewegung eines Gewebes in dem Objekt (11) basierend auf einer Zeit, die für eine Transmission und ein Empfangen der Ultraschallwelle zu dem Gewebe benötigt wird, zu bestimmen;
die Steuerungseinheit (8) ferner konfiguriert ist, die Bestrahlung mit dem Licht von der Lichtbestrahlungseinheit (3) zu ermöglichen, was durchzuführen ist, wenn bestimmt ist, dass die fotoakustische Messkopfeinheit in Kontakt mit dem Objekt (11) ist und sich das Gewebe in der Richtung hin zu der fotoakustischen Messkopfeinheit (1) bewegt oder stationär relativ zu der fotoakustischen Messkopfeinheit (1) ist;
die Steuerungseinheit (8) ferner konfiguriert ist, die Bestrahlung mit dem Licht von der Lichtbestrahlungseinheit (3) zu stoppen, was durchzuführen ist, wenn bestimmt ist, dass die fotoakustische Messkopfeinheit (1) nicht in Kontakt mit dem Objekt (11) ist, oder wenn bestimmt ist, dass die fotoakustische Messkopfeinheit (1) in Kontakt mit dem Objekt (11) ist und sich das Gewebe in der Richtung weg von der fotoakustischen Messkopfeinheit bewegt.

2. Objektinformationserhaltevorrichtung nach Anspruch 1, wobei die Lichtbestrahlungseinheit weiter außen als der Messkopf lokalisiert ist.

3. Objektinformationserhaltevorrichtung nach Anspruch 1, wobei der Messkopf weiter außen als die Lichtbestrahlungseinheit lokalisiert ist, und die Lichtbestrahlungseinheit ein Bestrahlungsende aufweist, das koplanar mit einer Ultraschallwellentransmissions- und -empfangsoberfläche des Messkopfs positioniert ist.

4. Objektinformationserhaltevorrichtung nach Anspruch 1, wobei eine Ultraschallwellentransmissions- und -empfangsoberfläche des Messkopfs mit einem akustischen Anpassmittel bereitgestellt ist, und ein Bestrahlungsende der Lichtbestrahlungseinheit in Kontakt mit dem akustischen Anpassmittel ist.

5. Objektinformationserhaltevorrichtung nach Anspruch 1, wobei die fotoakustische Messkopfeinheit ferner eine Lichtabschirmwand, die außen von dem Messkopf und der Lichtbestrahlungseinheit lokalisiert ist, enthält, und eine Ultraschallwellentransmissions- und -empfangsoberfläche des Messkopfs und ein Bestrahlungsende der Lichtbestrahlungseinheit weiter von dem Objekt entfernt sind als ein Ende der Lichtabschirmwand.

6. Objektinformationserhaltevorrichtung nach Anspruch 1, wobei die Steuerungseinrichtung konfiguriert ist, zu bestimmen, dass der Messkopf nicht akustisch an das Objekt angepasst ist, in einem Fall, bei dem die reflektierte Welle, die von einer Position, die mit einer Grenze zwischen der fotoakustischen Messkopfeinheit und dem Objekt zusammenfällt, erhalten wird, größer als ein Signal ist, das von dem Objekt in einem Zustand erhalten wird, in dem er akustisch angepasst ist.

7. Objektinformationserhaltevorrichtung nach Anspruch 1, wobei die Steuerungseinheit konfiguriert ist, zu bestimmen, dass der Messkopf akustisch an das Objekt angepasst ist, in einem Fall, in dem die reflektierte Welle, die von einer Position, die mit einem inneren Teil des Objekts zusammenfällt, erhalten wird, größer als Rauschen, das von dem Messkopf und dem Prozessor erzeugt wird, ist.

8. Objektinformationserhaltevorrichtung nach Anspruch 1, wobei die Steuerungseinheit konfiguriert ist, zu bestimmen, dass der Messkopf akustisch an das Objekt angepasst ist in einem Fall, in dem die reflektierte Welle, die von einer Position, die mit einem inneren Teil des Objekts zusammenfällt, erhalten wird, eine Frequenzkomponente, die verschieden von Frequenzkomponenten, die Rauschen, das von dem Messkopf und dem Prozessor erzeugt wird, entspricht, aufweist.

9. Objektinformationserhaltevorrichtung nach Anspruch 1,
wobei der Messkopf konfiguriert ist, die Ultraschallwelle wiederholt auf das Objekt zu transmittieren und die davon reflektierte Welle zu empfangen, und
die Steuerungseinheit konfiguriert ist, ein Signal entsprechend dem Gewebe innerhalb des Objekts von der reflektierten Welle zu extrahieren, und dann zu bestimmen, dass sich die fotoakustische Messkopfeinheit in der Richtung weg von dem Objekt bewegt in einem Fall, in dem eine längere Zeit für eine spätere Ultraschallwellentransmission und -empfang benötigt wird, um das so extrahierte Signal zu erhalten.

10. Objektinformationserhaltevorrichtung nach Anspruch 1, wobei die Steuerungseinheit konfiguriert ist, die Bestrahlung mit Licht von der Lichtbestrahlungseinheit in einem Fall zu stoppen, in dem die Messkopfeinheit und das Objekt sich relativ in einer Richtung weg voneinander bewegen, und eine Geschwindigkeit, mit der sich die Messkopfeinheit und das Objekt relativ voneinander fortbewegen, einen Schwellenwert überschreitet.

11. Objektinformationserhaltevorrichtung nach Anspruch 1, wobei die Lichtbestrahlungseinheit konfiguriert ist, die Lichtbestrahlung zu periodischen Bestrahlungszeiten durchzuführen.

12. Objektinformationserhaltevorrichtung nach Anspruch 1,
wobei die Lichtbestrahlungseinheit konfiguriert ist, das Licht von der Lichtquelle zu führen, um von einem Beleuchtungslichtemissionsende (3b) der Lichtbestrahlungseinheit das Objekt zu bestrahlen, und der Messkopf konfiguriert ist, an einer Empfangsoberfläche die fotoakustische Welle, die von dem Objekt, das mit dem Licht durch die Lichtbestrahlungseinheit bestrahlt wird, zu empfangen, und
wobei die fotoakustische Messkopfeinheit ferner eine Lichtabschirmwand, die außerhalb des Messkopfs und der Lichtbestrahlungseinheit lokalisiert ist, enthält,
das Beleuchtungslichtemissionsende der Lichtbestrahlungseinheit und die Empfangsoberfläche des Messkopfs auf einer gleichen Höhe sind; und
ein Ende der Lichtabschirmwand jenseits der Höhe des Beleuchtungslichtemissionsendes und der Empfangsoberfläche hervorragt, um eine Unebenheit auf einer Oberfläche der fotoakustischen Messkopfeinheit bereitzustellen, die das Beleuchtungslichtemissionsende und die Empfangsoberfläche aufweist.

13. Verfahren zum Steuern einer Objektinformationserhaltevorrichtung, die aufweist:
eine fotoakustische Messkopfeinheit (1) mit einer Lichtbestrahlungseinheit (3), die konfiguriert ist, Licht (13) von einer Lichtquelle (4) zu leiten, um ein Objekt (11) zu bestrahlen, und einem Messkopf (2), der konfiguriert ist, eine fotoakustische Welle, die von dem Objekt, das mit dem Licht durch die Lichtbestrahlungseinheit bestrahlt wird, zu erzeugen, wobei der Messkopf (2) konfiguriert ist, eine Ultraschallwelle auf das Objekt (11) zu transmittieren und eine reflektierte Welle der transmittierten Ultraschallwelle zu empfangen;
einen Prozessor, der konfiguriert ist, eine Bildinformation über einen inneren Teil des Objekts basierend auf der fotoakustischen Welle, die durch den Messkopf empfangen wird, zu erzeugen; und
eine Steuerungseinheit (8), die konfiguriert ist, die Bestrahlung mit dem Licht von der Lichtbestrahlungseinheit zu steuern,
**dadurch gekennzeichnet, dass**
das Verfahren die Schritte aufweist:
Bestimmen durch die Steuerungseinheit (8) unter Verwendung der reflektierten Welle, ob die fotoakustische Messkopfeinheit (1) in Kontakt mit dem Objekt (11) in solch einer Weise ist, dass der Messkopf (2) akustisch an das Objekt (11) angepasst ist, oder nicht;
Bestimmen einer Bewegung eines Gewebes in dem Objekt (11) durch die Steuerungseinheit (8) basierend auf einer Zeit, die für eine Transmission und ein Empfangen der Ultraschallwelle zu dem Gewebe benötigt wird;
Ermöglichen, durch die Steuerungseinheit (8), der Bestrahlung mit dem Licht von der Lichtbestrahlungseinheit (3), was durchzuführen ist, wenn bestimmt wird, dass die fotoakustische Messkopfeinheit in Kontakt mit dem Objekt (11) ist und sich das Gewebe in der Richtung hin zu der fotoakustischen Messkopfeinheit (1) bewegt oder stationär relativ zu der fotoakustischen Messkopfeinheit (1) ist;
Stoppen, durch die Steuerungseinheit (8), der Bestrahlung mit dem Licht von der Lichtbestrahlungseinheit (3), was durchzuführen ist, wenn bestimmt wird, dass sich die fotoakustische Messkopfeinheit nicht in Kontakt mit dem Objekt (11) befindet, oder wenn bestimmt wird, dass sich die fotoakustische Messkopfeinheit (1) in Kontakt mit dem Objekt (11) befindet und sich das Gewebe in der Richtung weg von der fotoakustischen Messkopfeinheit bewegt.

## Revendications

1. Appareil d'acquisition d'informations d'objet, comprenant :
une unité sonde photo-acoustique (1) comprenant une unité d'exposition à un rayonnement de lumière (3) configurée pour guider une lumière (13) à partir d'une source de lumière (4) pour exposer un objet (11) à un rayonnement, et une sonde (2) configurée pour recevoir une onde photo-acoustique générée à partir de l'objet exposé au rayonnement de la lumière par l'unité d'exposition à un rayonnement de lumière, la sonde (2) étant configurée pour transmettre une onde ultrasonore en direction de l'objet (11) et pour recevoir une onde réfléchie de l'onde ultrasonore transmise ;
un processeur configuré pour créer des informations d'image concernant une partie interne de l'objet sur la base de l'onde photo-acoustique reçue par la sonde ; et
un organe de commande (8) configuré pour commander l'exposition au rayonnement de la lumière à partir de l'unité d'exposition à un rayonnement de lumière,
**caractérisé en ce que**
l'organe de commande (8) est configuré pour déterminer si l'unité sonde photo-acoustique (1) est, ou non, en contact avec l'objet (11) de sorte que la sonde (2) soit acoustiquement appariée à l'objet (11) au moyen de l'onde réfléchie ;
l'organe de commande (8) est en outre configuré pour déterminer un déplacement d'un tissu dans l'objet (11) sur la base d'un temps nécessaire à une transmission de l'onde ultrasonore vers le tissu et à une réception de ladite onde par ce dernier ;
l'organe de commande (8) est en outre configuré pour permettre une exécution de l'exposition au rayonnement de la lumière à partir de l'unité d'exposition à un rayonnement de lumière (3) lorsqu'il est déterminé que l'unité sonde photo-acoustique est en contact avec l'objet (11) et que le tissu se déplace dans le sens allant vers l'unité sonde photo-acoustique (1) ou qu'il est fixe par rapport à l'unité sonde photo-acoustique (1) ;
l'organe de commande (8) est en outre configuré pour interrompre l'exécution de l'exposition au rayonnement de la lumière à partir de l'unité d'exposition à un rayonnement de lumière (3) lorsqu'il est déterminé que l'unité sonde photo-acoustique (1) n'est pas en contact avec l'objet (11), ou lorsqu'il est déterminé que l'unité sonde photo-acoustique (1) est en contact avec l'objet (11) et que le tissu se déplace dans le sens allant en s'éloignant de l'unité sonde photo-acoustique.

2. Appareil d'acquisition d'informations d'objet selon la revendication 1, dans lequel l'unité d'exposition à un rayonnement de lumière est située plus à l'extérieur que la sonde.

3. Appareil d'acquisition d'informations d'objet selon la revendication 1, dans lequel la sonde est située plus à l'extérieur que l'unité d'exposition à un rayonnement de lumière, et l'unité d'exposition à un rayonnement de lumière comporte une extrémité d'exposition à un rayonnement positionnée dans le même plan qu'une surface de transmission et de réception d'onde ultrasonore de la sonde.

4. Appareil d'acquisition d'informations d'objet selon la revendication 1, dans lequel une surface de transmission et de réception d'onde ultrasonore de la sonde est pourvue d'un agent d'appariement acoustique, et une extrémité d'exposition à un rayonnement de l'unité d'exposition à un rayonnement de lumière est en contact avec l'agent d'appariement acoustique.

5. Appareil d'acquisition d'informations d'objet selon la revendication 1, dans lequel l'unité sonde photo-acoustique comprend en outre une paroi de protection contre la lumière située à l'extérieur de la sonde et de l'unité d'exposition à un rayonnement de lumière, et une surface de transmission et de réception d'onde ultrasonore de la sonde et une extrémité d'exposition à un rayonnement de l'unité d'exposition à un rayonnement de lumière sont positionnées plus éloignées de l'objet qu'une extrémité de la paroi de protection contre la lumière.

6. Appareil d'acquisition d'informations d'objet selon la revendication 1, dans lequel l'organe de commande est configuré pour déterminer que la sonde n'est pas acoustiquement appariée à l'objet dans un cas dans lequel l'onde réfléchie acquise depuis une position coïncidant avec une limite entre l'unité sonde photo-acoustique et l'objet a une intensité supérieure à un signal acquis à partir de l'objet dans un état d'appariement acoustique.

7. Appareil d'acquisition d'informations d'objet selon la revendication 1, dans lequel l'organe de commande est configuré pour déterminer que la sonde est acoustiquement appariée à l'objet dans un cas dans lequel l'onde réfléchie acquise depuis une position coïncidant avec une partie interne de l'objet a une intensité supérieure à des bruits générés à partir de la sonde et du processeur.

8. Appareil d'acquisition d'informations d'objet selon la revendication 1, dans lequel l'organe de commande est configuré pour déterminer que la sonde est acoustiquement appariée à l'objet dans un cas dans lequel l'onde réfléchie acquise depuis une position coïncidant avec une partie interne de l'objet contient une composante de fréquence autre que des composantes de fréquence correspondant à des bruits générés à partir de la sonde et du processeur.

9. Appareil d'acquisition d'informations d'objet selon la revendication 1,
dans lequel la sonde est configurée pour transmettre l'onde ultrasonore de façon répétée en direction de l'objet et pour en recevoir l'onde réfléchie, et
l'organe de commande est configuré pour extraire un signal correspondant au tissu intérieur à l'objet à partir de l'onde réfléchie, et pour déterminer ensuite que l'unité sonde photo-acoustique se déplace dans le sens qui va en s'éloignant de l'objet dans un cas dans lequel un temps plus long est nécessaire, par rapport aux dernières transmission et réception d'onde ultrasonore, pour acquérir le signal ainsi extrait.

10. Appareil d'acquisition d'informations d'objet selon la revendication 1, dans lequel l'organe de commande est configuré pour interrompre l'exposition à un rayonnement de lumière à partir de l'unité d'exposition à un rayonnement de lumière dans un cas dans lequel l'unité sonde et l'objet se déplacent relativement dans une direction les éloignant l'un de l'autre et dans lequel une vitesse à laquelle l'unité sonde et l'objet se déplacent relativement dans une direction les éloignant l'un de l'autre dépasse une valeur seuil.

11. Appareil d'acquisition d'informations d'objet selon la revendication 1, dans lequel l'unité d'exposition à un rayonnement de lumière est configurée pour exécuter l'exposition au rayonnement de la lumière à des temporisations d'exposition à un rayonnement périodique.

12. Appareil d'acquisition d'informations d'objet selon la revendication 1,
dans lequel l'unité d'exposition à un rayonnement de lumière est configurée pour guider la lumière à partir d'une source de lumière pour exposer à un rayonnement, à partir d'une extrémité d'émission de lumière d'éclairement (3b) de l'unité d'exposition à un rayonnement de lumière, l'objet, et la sonde est configurée pour recevoir, au niveau d'une surface de réception, l'onde photo-acoustique générée à partir de l'objet exposé au rayonnement de la lumière par l'unité d'exposition à un rayonnement de lumière, et
dans lequel l'unité sonde photo-acoustique comprend en outre une paroi de protection contre la lumière située extérieurement à la sonde et à l'unité d'exposition à un rayonnement de lumière,
l'extrémité d'émission de lumière d'éclairement de l'unité d'exposition à un rayonnement de lumière et la surface de réception de la sonde sont situées à la même hauteur ; et
une extrémité de la paroi de protection contre la lumière dépasse la hauteur de l'extrémité d'émission de lumière d'éclairement et la surface de réception de façon à établir une irrégularité sur une surface de l'unité sonde photo-acoustique comprenant l'extrémité d'émission de lumière d'éclairement et la surface de réception.

13. Procédé pour commander un appareil d'acquisition d'informations objet comportant :
une unité sonde photo-acoustique (1) comprenant une unité d'exposition à un rayonnement de lumière (3) configurée pour guider une lumière (13) à partir d'une source de lumière (4) pour exposer un objet (11) à un rayonnement, et une sonde (2) configurée pour recevoir une onde photo-acoustique générée à partir de l'objet exposé au rayonnement de la lumière par l'unité d'exposition à un rayonnement de lumière, la sonde (2) étant configurée pour transmettre une onde ultrasonore en direction de l'objet (11) et pour recevoir une onde réfléchie de l'onde ultrasonore transmise ;
un processeur configuré pour créer des informations d'image concernant une partie interne de l'objet sur la base de l'onde photo-acoustique reçue par la sonde ; et
un organe de commande (8) configuré pour commander l'exposition au rayonnement de la lumière à partir de l'unité d'exposition à un rayonnement de lumière,
le procédé étant **caractérisé en ce qu'**il comprend les étapes consistant à :
déterminer, par l'organe de commande (8), si l'unité sonde photo-acoustique (1) est, ou non, en contact avec l'objet (11) de sorte que la sonde (2) soit acoustiquement appariée à l'objet (11) au moyen de l'onde réfléchie ;
déterminer, par l'organe de commande (8), un déplacement d'un tissu dans l'objet (11) sur la base d'un temps nécessaire à une transmission de l'onde ultrasonore en direction du tissu et à une réception de cette dernière ;
permettre l'exécution, par l'organe de commande (8), de l'exposition au rayonnement de lumière à partir de l'unité d'exposition à un rayonnement de lumière (3) lorsqu'il est déterminé que l'unité sonde photo-acoustique est en contact avec l'objet (11) et que le tissu se déplace dans un sens allant vers l'unité sonde photo-acoustique (1) ou qu'il est fixe par rapport à l'unité sonde photo-acoustique (1) ;
interrompre l'exécution, par l'organe de commande (8), de l'exposition à un rayonnement de la lumière à partir de l'unité d'exposition à un rayonnement de lumière (3) lorsqu'il est déterminé que l'unité sonde photo-acoustique n'est pas en contact avec l'objet (11), ou lorsqu'il est déterminé que l'unité sonde photo-acoustique (1) est en contact avec l'objet (11) et que le tissu se déplace dans le sens allant en s'éloignant de l'unité sonde photo-acoustique.
